# EUROPEAN PATENT APPLICATION

(11) **EP 2 460 836 A1**
(43) Date of publication of application: **06.06.2012**
(21) Application number: 10803750.8
(22) Date of filing: 30.07.2010
(51) Int. Cl.: C08G 18/08, C08G 63/08, C08G 63/82, A61L 27/18, A61L 27/34

(54) **PROCESS FOR PRODUCING BIOPOLYMER MEMBRANES AND BIOPOLYMER MEMBRANES PRODUCED BY THIS PROCESS**

(30) Priority: 31.07.2009 BR PI0902480
(71) Applicant: União Brasileira de Educação e Assistência - Mantenedora da PUC RS, Partenon, Porto Alegre, RS (BR); A.S. Technology Componentes Especiais, Dos Campos, São Paulo (BR)
(72) Inventor: LIGABUE, Rosane Angélica, RS (BR); EINLOFT, Sandra, CEP: 91720-060 - Porto Alegre RS (BR); BRAGA DA SILVA, Jefferson, CEP: 90470-150 - Petrópolis Porto Alegre (BR); POLTRONIERI, Tassiani, CEP: 93330-110 - Novo Hamburgo (BR); DE LIMA DULLIUS, Jeane Estela, CEP: 96745-000 Charqueadas (BR); VIEZZER, Christian, CEP: 91050-000 Porto Alegre (BR); CANTARELLI, Denise, CEP: 90110-170 - Porto Alegre (BR); DALOSTO JAHNO, Vanusca, CEP: 91510-310 - Porto Alegre (BR)
(74) Representative: Pulieri, Gianluca Antonio
(86) International application number: PCT/BR2010/000252
(87) International publication number: WO 2011/011846

(57) **Abstract**

The present invention describes the process of producing biopolymeric membranes and the membranes obtained by this process. Particularly, the biopolymeric membranes of the present invention comprise poly (urethane-caprolactone) and can be used for nerve and bone regeneration.

## Description

### Field of the Invention

The present invention describes the process of producing biopolymeric membranes and the membranes obtained by this process. Particularly, the biopolymeric membranes of the present invention comprise poly(urethane-caprolactone) and can be used for nerve and bone regeneration. The present invention chiefly concerns the fields of medicine, chemistry and tissue engineering.

### Background of the Invention

The development of biodegradable and bioreabsorbable polymers has received much attention in recent years, as these polymers have broad application in the environmental and biomedical sector, such as, implant devices, catheterism devices, among others, and is also promising in the field of tissue engineering (Grad, et. al. 2003).

Biomaterials have shown a growth rate of 11% per year, which demonstrates the major interest and need for this kind of product (Mirtchi, et. al. 1989).

Biodegradable polyurethanes are formed from aliphatic diisocyanates having different polyols, polyethylene adipate and poly(caprolactone), and chain extenders such as diols, diamines and disulfates (Hori, et. al.).

This work presents the synthesis, characterization and in vitro evaluation of polyurethane for use as biopolymers (BPU), particularly in the form of biocompatible membranes.

In the realm of patents, some documents describe materials comprising polyurethanes and their use.

Document US 5, 151, 315 describes composites having medical use, preferably orthopedic, comprising mixtures of polycaprolactone and polyurethane. Particularly, this material comprises from 20 to 70% b/w of polyester caprolactone polyurethane, 80 to 30% by weight of polycaprolactone and a cover on the center, which has three layers. These mixtures are elastic when heated. The present invention differs from this document in that it does not comprise covers as in said document, and the membrane is formed by a uniform layer of polyurethanes, such as polyurethane caprolactone.

Document WO 2005/111110 describes non-toxic biodegradable polyurethanes for controlled release of drugs for tissue regeneration comprising polyurethane formed by polyoxyethylated copolymers, in particular triblocks resulting in the combination Polyethyleneglycol-IA-caprolactone, CL-PEG-C L . T h e incorporation of other aminoacids in chain IA if made by way of polyisocyanate or chain extender. The present invention differs from this document because it does not need natural aminoacids and because it comprises the dissolution of the polymer in eluents, especially THF, a fact that is not described in said document, forming the biocompatible membrane of the present invention.

Document US 2008/0262613 describes biocompatible and biodegradable polyurethane materials comprising linear segment of polyurethanes or polyurethanes with cross links containing polyols, diisocyanates and chain extenders. The present invention differs from this document by comprising the dissolution of the polymer in eluents, especially THF, a fact that is not described in said document, to form the biocompatible membrane of the present invention.

The article by Hong (2007) describes the synthesis of the poly (3-caprolactone-co-b-butyrolactone) (PCLBL)-base polyurethane (PCLBL-PU) and the degradation of this polymer was studied. The present invention differs from this document by additionally comprising the dissolution of the polymer in THF to form a membrane destined for use in the medical sector and by not containing butyrolactone in its formula.

No documents anticipating or suggesting the teachings of the present invention were found in the literature researched, to the extent that the solution now proposed bears novelty and inventive activity in light of the state of the art.

### Summary of the Invention

In one aspect, the present invention provides a synthesis of polymers for use as biopolymers (BPU), especially in the form of biocompatible membranes.

It is an object of the present invention to provide a process of producing biopolymeric membranes that comprises the steps of:
a) dissolving at least one polyol in solvent;
b) adding a mixture of isocyanate and catalyst;
c) eliminating the solvent;
d) solubilizing the polymer obtained in eluents and adjusting the thickness.

Particularly, the PU-PCL films were prepared by way of a solution with 20% of PU-PCL in tetrahydrofuran (THF) and these were poured onto a glass plate, on which there was placed the polymeric solution, a 100 micrometer strain gauge was placed, and the films were vacuum-dried for total withdrawal of the solvent.

Particularly, the reactions are carried out in a reactor, under constant mechanical agitation and temperature of the reaction system.

Particularly, the addition of isocyanates optionally comprises the addition of chain extenders. In a preferred embodiment, the reaction system used PLLA as polyol, HDI as isocyanate and 1,4-butanodiol as chain extender.

In an optional embodiment, the reaction system used PCL as polyol and HDI as isocyanate.

In a second aspect, the present invention provides biopolymeric membranes, capable of interacting with biological materials acting, for example, as cell growth matrix.

It is therefore an object of the present invention to provide polymeric membranes obtained by the membrane production process described above.

These and other objects of the invention will be appreciated and better understood in the detailed description of the invention.

### Brief Description of the Drawings

Figure 1 shows the spectrum of the biopolymer BPU1.

Figure 2 shows the fibroblasts on the biopolymeric membrane of the present invention.

### Detailed Description of the Invention

The examples shown here are only intended to exemplify one of the innumerous ways of carrying out the invention, but without limiting the scope thereof. Solvent

The solvent of the present invention comprises the group of aprotic solvents such as, but not limited to, acetone or dichloromethane. These solvents have major dipole moments and preferably solvate species positively charged via their negative dipoles, favoring the Sn2 reaction mechanisms. Particularly, the present invention uses acetone to solubilize the polyol.

### Polyols

The polyol of the present invention was chosen from the group that comprises polyhydroxyacids, such as polylactides and polyglucosides, polyethylene adipate and poly(caprolactones). Particularly, in the present invention polycaprolactone (PCL) is used.

### Isocyanates

The isocyanates of the present invention are chosen from the group that comprises aromatic, aliphatic, cycloaliphatic and/or polycyclic isocyanates, allowing the achievement of an infinite variety of compounds with different physical and chemical properties. There are various diisocyanates and triisocyanates, such as aliphatic diisocyanates. Particularly, the present invention uses hexamethylene diisocyanate (HDI). Particularly, the calculations of the ideal quantity of polyols and HDI are in molar ratio, and the isocyanate/polyol ratio varies from 2:1 to 0.5:1, preferably the rate used was 1.2:1.

### Catalyst

The catalysts of the present invention can be chosen from among catalysts known in the state of the art, including, but not limited to, tin dibutyl dilaurate (DBTDL). Particularly, the present invention uses 0.1% of DBTDL.

### Eluents

The eluents used in the present invention comprise, but are not limited to, different groups of compounds such as, for example, ketones, acetone, methyl iso-butyl-ketone - MIBK, methyl ethyl ketone - MEK, ether, tetrahydrofurane - THF, alcohol, tert-butyl alcohol - TBA, methylene chloride, trichloroethylene, dioxane, ethyl acetate and isobutyl acetate. Particularly, the present invention uses THF.

### THF

Tetrahydrofurane or THF is a heterocyclic organic compound used as eluent. It is ether, polar, and can be obtained by hydrogenating the furan. The present invention uses 20% of polymeric solution in THF to dissolve the polymer.

### Process of producing biopolymeric membranes

The process of producing biopolymeric membranes comprises the steps of:
a) dissolving at least one polyol in solvent;
b) adding a catalyst to this solution;
c) adding an isocyanate;
c) eliminating the solvent;
d) solubilizing the polymer obtained in eluents and adjusting the thickness.

The reactions are carried out in a reactor, under constant mechanical agitation and temperature of the reaction system.

The step of preparing the films consists of solubilizing the polymer obtained and adjusting the thickness thereof with the help of a suitable instrument, such as, for example, a 100 micrometer strain gauge, followed by vacuum drying for the total withdrawal of the solvent, forming the dry films.

The atmosphere of the reactor is an inert atmosphere, to avoid secondary reactions of the reagents and to increase the production yield of the polymer. Particularly, the reactions were carried out in an inert atmosphere of N2, with mechanical agitation, and constant temperature of the reaction system. The content of free NCO was accompanied by titration and infra-red spectroscopy.

Particularly, the molar ratio of isocyanate/polyol varies from 2:1 to 0.5:1, preferably the ratio used was 1.2:1, the temperature of the reaction system PCL/HDI (BPU2) at 60°C.

### Polyurethanes

The polyurethanes (PUs) used as biomaterial have a biocompatibility character and physical and mechanical characteristics that allows them to be used in implant devices such as intra-aortic balloon, breast implants, angioplasty balloons, catheterism devices, among others. Biodegradable polyurethanes can be formed from diisocyanates with different polyols and chain extenders. The characteristics of the polyurethane formed in the present invention will depend on the polyol and the diisocyanate used.

### Biopolymeric Membrane obtained

A biopolymeric membrane obtained by the process of producing biopolymeric membranes. Particularly, these membranes are 100µm to 5mm in thickness. These membranes were used for the in vivo and in vitro tests carried out with osteoblast cells, in which the biocompatibility of the polymer was confirmed. Particularly, the 100µm PU-PCL films were sterilized by ethylene oxide by the company Esteriliplus -Esterilização à Óxido de Etileno Ltda., in order to be used in surgeries.

### Example 1 - Preferred Embodiment

### Production of the polymer

This work presents the synthesis of polyurethane biomaterials using different polyols, with the aim of comparing the characteristics that these reagents confer to the synthesized bioPU. The materials used in the syntheses were: hexamethylene diisocyanate (HDI), poly-(caprolactone) diol (PCL), the chain extender 1,4-butanodiol and the catalyst tin dibutyl dilaurate (DBTDL). All the reactions were carried out under inert atmosphere of N2, with mechanical agitation and the molar ratio of NCO/OH used was 1.2. The temperature of the system PCL/HDI (BPU) was kept at 60°C. The content of free NCO in the reaction was accompanied by titration with N-dibutylamine and Infra-red Spectroscopy (IV), where a decrease was noted in the band relating to the diisocyanate NCO (≈ 2270 cm-1). The BPU has molar mass 120359 g/mol and IP 1.5, and this data was obtained by Gel Permeation Chromatography (GPC), as described in literature. The synthesized bioPU was also characterized by IV. The spectrum of BPU presented a band at 1731 cm-1 characteristic of urethane C=O stretching. At 1530 cm-1 a band characteristic of urethane C-N and N-H was noted, and at 3383 cm-1 the spectrum presents a band characteristic of N-H. At about 2940 cm-1 a band characteristic of C-H stretching is noted. These attributions are in accordance with that described in literature. To verify the degradation in aqueous medium (distilled water, pH 6.3), a BPU sample was placed in 150 mL of distilled water, and was left for 15 days. In the BPU there was a decrease in the pH to 5.0 in 8 days. This result indicates a slow and stable degradation of the BPU, since it derives from a polyol caprolactone, where the lactone group has greater resistance to hydrolysis than an ester group.

The synthesis of polyurethane (PU) from hexamethylene diisocyanate (HDI), polyol poly-(caprolactone) diol, can be used as a cell growth matrix. The formation reaction of the polyurethane was accompanied by consumption of the diisocyanate group over time and it was characterized by the IV and GPC technique presenting an average molar mass of 120,359g/mol. The degradation of the PU synthesized in an aqueous medium was studied, and it was noted that the degradation process began as of the 8th day. A preliminary in vitro evaluation was also made using fibroblast cells of mice (NIH3T3).

### Materials

The materials used in the synthesis were hexamethylene diisocyanate (HDI), polyol poly-(caprolactone) diol (PCL, Mn 2000g/mol), and the catalyst tin dibutyl dilaurate (DBTDL). The reaction was carried out at 60°C under inert atmosphere of N2, with mechanical agitation and molar ratio of NCO/OH of 1.2. The content of free NCO in the reaction was accompanied by titration with N-dibutylamine and Infra-red Spectroscopy using Perkin Elmer Instruments Spectrum One FT-IR Spectrometer equipment, wherein a decrease in the band relating to the Diisocyanate NCO (≈ 2270cm-1) was noted. The Gel Permeation Chromatography (GPC) analyses were carried out with a 1515 isocratic HPLC pump using the refractive index detector Waters Instruments 2412 and THF as eluent. To verify degradation in the aqueous medium (distilled water, pH 6.3) a sample of BPU (2.8956g) was placed in 150 mL of distilled water, and was left for 16 days. This Degradation Test was made by evaluating the pH variation in the medium over time, using a Digimed DM-20 pHmeter, which was calibrated with Quimis calibration solutions pH 4.01 and 6.86. For the in vitro evaluation, 0.5x105 fibroblast cells (NIH3T3) were kept in culture on the surface of the BPU, in D-MEM medium supplemented with 10% bovine fetal serum and antibiotics, under a humid atmosphere with 5% of CO2. Cellular adhesion was verified by fixing the species in methanol and coloring them with hematoxylin and eosin (HE).

### Results and Discussion

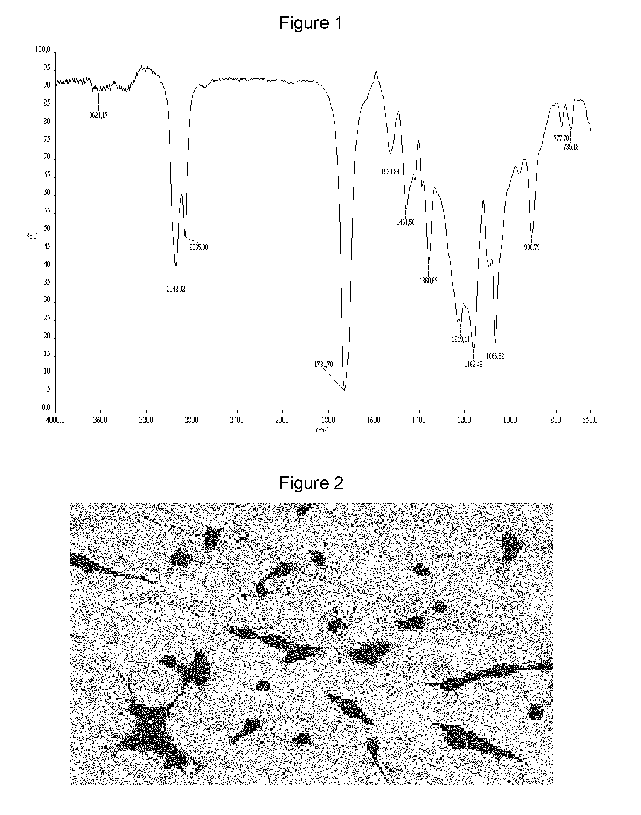

The polyurethane obtained from the PCL (BPU) presented a molar mass of 120.359 g/mol and polydispersity of 1.5. The characterization of the BPU by Infra-red Spectroscopy (FT-IR) provided a spectrum which presented a band at 1731 cm-1 characteristic of C=O of the urethane group. At 1530 cm-1 a band relating to the urethane groups C-N and N-H was noted and at about 3383 cm-1 the spectrum presented a band characteristic of group N-H (Fig.1), in accordance with data from literature. The Degradation Test reveals that the pH of the medium begins to decline reaching a value of 5.0 on the 8th day. Coloring by the HE method demonstrates that the polymer supports adhesion and proliferation of the NIH3T3 fibroblast cells (Fig. 2). Since they can only proliferate when they adhere to the surface, whereby secreting proteins from the extracellular matrix continuing with its program.

By using the infra-red technique, it was possible to verify the formulation of the desired polyurethane, which was confirmed by the attributions of the bands in relation to data from literature. The degradation test in aqueous medium showed a slow and stable degradation of the BPU, since it derives from a caprolactone polyol, where the lactone group has a certain resistance to hydrolysis. Cellular adhesion on the surface of the polymer can be confirmed by coloring the fibroblast cells with hematoxylin and eosin.

Therefore, the present invention relates to a poly(urethane-caprolactone) based polymeric material, PU-PCL, for nerve and bone regeneration. The polymer was obtained by the production process described previously. The polymer obtained was dissolved in THF to form films having a thickness of 100 µm that are used for the in vivo tests and for disks used in the in vitro tests. The in vivo tests were carried out on a group of Wistar mice on which a piece of polymer was inserted into the back, sciatic nerve, muscle and bone tissue. A time accompaniment was carried out to evaluate a possible inflammatory response. The in vitro tests were carried out with osteoblast cells in which the biocompatibility of the polymer was confirmed.

Those skilled in the art will appreciate the knowledge presented herein and may reproduce the invention in the embodiments set forth and in other variants, encompassed within the scope of the accompanying claims.

## Claims

1. A production process of polymeric membranes **characterized by** comprising the steps of:
a) dissolving at least one polyol in solvent;
b) adding a catalyst to this solution;
c) adding an isocyanate;
c) eliminating the solvent;
d) solubilizing the polymer obtained in eluents and adjusting the thickness.

2. A production process, according to claim 1, **characterized** wherein the polyol is polycaprolactone (PCL).

3. A production process, according to claim 1, **characterized** wherein the solvent is ketones having low boiling point (acetone and MEK).

4. A production process, according to claim 1, **characterized** wherein the isocyanate is hexamethylene diisocyanate (HDI).

5. A production process, according to claim 1, **characterized by** using 0.1% of catalyst.

6. A production process, according to claim 1, **characterized** wherein the catalyst is made of tin (dibutyl dilaurate).

7. A production process, according to claim 1, **characterized by** optionally comprising chain extenders.

8. A production process, according to claim 7, **characterized** wherein the chain extender is 1,4-butanodiol.

9. A production process, according to claim 1, **characterized by** using 20% of polymeric solution in eluent.

10. A production process, according to claim 9, **characterized** wherein the eluent is THF.

11. A production process, according to claim 1, **characterized** wherein the reaction system PCL/HDI (BPU) allows a polymer to be obtained with a molar mass 120359 g/mol and Polydispersity Index of 1.5.

12. A production process, according to claim 1, **characterized** wherein said process is carried out in a reactor, under constant mechanical agitation and temperature of the reaction system.

13. A production process, according to claim 13, **characterized** wherein the temperature of the system PCL/HDI is kept at 60°C.

14. A production process, according to claim 1, **characterized** wherein the molar ratio of isocyanate/polyol Varies from 2:1 to 0.5:1.

15. A production process, according to claim 16, **characterized** wherein the molar ratio of isocyanate/polyol is 1.2:1.

16. A polymeric membrane **characterized** wherein the polyol polymer is polycaprolactone (PCL) with hexamethylene diisocyanate (HDI), being from 100µm to 5mm in thickness and being used for cellular regeneration, nerve regeneration and/or bone regeneration.
